(19) Europäisches Patentamt
European Patent Office
Office européen des brevets

(11) **EP 2 067 451 B1**

(12) **EUROPÄISCHE PATENTSCHRIFT**

(45) Veröffentlichungstag und Bekanntmachung des Hinweises auf die Patenterteilung:
**20.11.2013 Patentblatt 2013/47**

(51) Int Cl.:
**A61C 1/00** *(2006.01)* **A61C 19/00** *(2006.01)*

(21) Anmeldenummer: **09151926.4**

(22) Anmeldetag: **04.06.2002**

(54) **Medizinisches oder dentalmedizinisches Instrument und/oder Versorgungsgerät und/oder Pflegegerät und/oder System für das medizinische oder dentalmedizinische Instrument**

Medical or dental instrument and/or care device and/or system for the medical or dental instrument

Instrument médical ou médical dentaire et/ou appareil d'alimentation et/ou appareil de soin et/ou système destinés à l'instrument médical ou médical dentaire

(84) Benannte Vertragsstaaten:
**AT CH DE FI IT LI**

(30) Priorität: **07.06.2001 DE 10127772**

(43) Veröffentlichungstag der Anmeldung:
**10.06.2009 Patentblatt 2009/24**

(62) Dokumentnummer(n) der früheren Anmeldung(en) nach Art. 76 EPÜ:
**02748733.9 / 1 392 193**

(73) Patentinhaber: **Kaltenbach & Voigt GmbH**
**88400 Biberach (DE)**

(72) Erfinder:
• **Beier, Stefan**
**88400, Biberach (DE)**
• **Eibofner, Eugen**
**88400, Biberach-Mettenberg (DE)**
• **Heckenberger, Hans**
**88433 Aßmannshardt (DE)**
• **Strohmaier, Ernst**
**88427, Bad Schussenried (DE)**
• **Imhof, Armin**
**88400, Biberach (DE)**

(74) Vertreter: **Thun, Clemens**
**Mitscherlich & Partner**
**Patent- und Rechtsanwälte**
**Sonnenstrasse 33**
**80331 München (DE)**

(56) Entgegenhaltungen:
WO-A-98/06338      DE-A- 3 726 262
DE-A1- 19 729 177   US-A- 5 807 521
US-A- 6 092 722     US-A- 6 117 285

**Beschreibung**

Die Erfindung betrifft ein System zum Benutzen und Pflegen eines medizinischen oder dentalmedizinischen Instruments nach dem Oberbegriff des Anspruchs 1.

Bei einem medizinischen oder dentalmedizinischen Instrument handelt es sich um einen Gegenstand, der zur Bearbeitung des menschlichen oder tierischen Körpers oder Teilen (Prothesen) davon eingesetzt wird und dabei in Kontakt mit dem Körper kommt und dabei verunreinigt und/oder mit Krankheitserregern kontaminiert werden kann. Dies insbesondere dann, wenn das Instrument mit einem Sekret oder einer Flüssigkeit des Körpers in Kontakt kommt. Es ist deshalb erforderlich, nach der Bearbeitung eines Patienten bzw. vor der Bearbeitung eines nächsten Patienten das Instrument zu reinigen und/oder zu desinfizieren und/oder sterilisieren. Die jeweilige Abnahme ist davon abhängig, zum einen wie intensiv der Kontakt des Instruments mit dem Körper war und zum anderen welche Bearbeitungsmaßnahme durchgeführt worden ist.

Die Ausgestaltung und Funktion eines vorliegenden Instruments kann außerdem sehr unterschiedlich sein. Insbesondere bei einem vorgenannten Instrument, dessen Werkzeug durch einen Antrieb bewegt wird, bedarf es in gewissen Zeitabständen einer mechanischen Wartung des wenigstens einen Instruments, um dessen Funktionsfähigkeit aufrecht zu erhalten und Verschleiß und Funktionsstörungen zu vermeiden. Es gibt Instrumente, bei denen unabhängig davon, ob sie im Funktionsbetrieb verunreinigt und/oder kontaminiert werden, die Vorschrift besteht, das Instrument in gewissen Zeitabständen, z. B. nach bestimmten Betriebsstunden, zu warten. Die vorbeschriebenen Maßnahmen zur Aufrechterhaltung der Einsatzbereitschaft des wenigstens einen Instruments werden im Folgenden mit dem Begriff "Pflege" bezeichnet. Es gibt deshalb für ein vorliegendes Instrument eine Benutzungsphase, in der es auf den Körper wenigstens zeitweise einwirkt, und eine Pflegephase, in der auf das Instrument zur Aufrechterhaltung seiner Einsatzbereitschaft eingewirkt wird.

In der Benutzungsphase ist das Instrument mit einem medizinischen oder dentalmedizinischen Versorgungsgerät verbunden. Gemäß dem derzeitigen Standard weist das Versorgungsgerät eine elektronische Steuereinrichtung auf, von der sich eine biegsame bzw. flexible Versorgungsleitung erstreckt, mit der das Instrument durch eine lösbare Kupplung verbunden ist. Das Instrument bzw. sein Werkzeug wird für seine Funktion durch die Versorgungsleitung hindurch vom Versorgungsgerät versorgt bzw. gesteuert. Hierbei kann es sich um die Zuführung von Antriebsenergie und/oder Bearbeitungsmitteln handeln.

Dem Versorgungsgerät kann nur ein oder können mehrere unterschiedliche Instrumente zugeordnet sein, die wahlweise durch die Kupplung mit der Versorgungsleitung verbindbar sind. Hierbei können je nach Art des Instruments unterschiedliche Antriebsmittel vorhanden sein bzw. zugeführt werden. Ein Versorgungsgerät dieser Art ist z. B. in der DE 4 410 276 A1 beschrieben.

In der DE 42 20 522 A1 ist ein System zur Kenntlichmachung verschiedener Verbraucher, beispielsweise zahnärztliche Instrumente, gegenüber einer zu einem zahnärztlichen Behandlungsplatz gehörenden Energie-Versorgungseinheit beschrieben, mit an einer Versorgungsleitung vorgesehenen Codierungsmitteln zur Identifizierung eines der Versorgungsleitung zugeordneten Instruments und mit einer zu der Energie-versorgungseinheit gehörenden Auswerteschaltung, die durch elektrische Verbindungsleitungen mit den Codierungsmitteln verbunden ist. Die elektrischen Verbindungsleitungen erstrecken sich durch die Versorgungsleitung.

Aus der DE 19 729 177 A1 ist ein ärztliches Versorgungsgerät zu entnehmen, bei dem mit Hilfe einer elektronischen Steuereinrichtung ein an einem Antriebsmotor ankoppelbares Instrument mit Werkzeug versorgt und gesteuert werden kann. In dem Steuergerät sind herstellerseitig fest vorgegebene Betriebsprogramme für den Betrieb bestimmter Instrumente und Werkzeuge gespeichert. Zudem können benutzerspezifische Betriebsprogramme erstellt werden. Des weiteren kann mit Hilfe einer Auswahltaste ein Einstellmodus abgerufen werden, in dem der Benutzer beliebig in den Betriebsablauf eingreifen und die Betriebsparameter beliebig verändern kann.

In der DE 199 13 962 A1 ist eine Pflegevorrichtung für zahnärztliche Handstücke beschrieben, mit einem Gehäuse, das einen Pflegeraum umschließt. Im Pflegeraum ist wenigstens ein Kupplungsteil zur Aufnahme des einen Endes eines Instruments angeordnet, wobei sich eine Pflege-Zuführungsleitung zum Kupplungsteil erstreckt. Zwecks Gewährleistung einer einfachen Ausgestaltung und einer sicheren Funktion ist dem Kupplungsteil ein Sensor zugeordnet, der durch das Instrument in dessen Pflegestellung zwecks Abgabe eines Signals an eine Steuereinrichtung des Pflegegeräts aktiviert wird, worauf die Steuereinrichtung das Zuführungssystem für Pflegemittel ansteuert.

Eine Codierung an einem vorliegenden Instrument kann Identifizierungs- und/oder Betriebsparameterdaten enthalten und stellt somit ein Speicherelement für solche Daten dar, die bei einer Kopplung des Instruments mit einem medizinischen oder dentalmedizinischen Versorgungsgerät vom Gerät abrufbar sind und zur Steuerung der Versorgung des Instruments benutzt werden können.

Aus der WO 98/06338 A2 ist ein System zum Benutzen und Pflegen eines medizinischen Instruments bekannt. Das System umfasst ein Bediengerät, von dem das Instrument für die Benutzung mit Wasser und Strom versorgt wird. In dem Instrument sind Betriebsparameter wie Temperatur- und Stromwerte gespeichert, die von einer Lesevorrichtung im Bediengerät ausgelesen werden können. Das Bediengerät steht mit einem Computer in Datenaustausch.

Der Erfindung liegt die Aufgabe zugrunde, ein System zum Benutzen und Pflegen eines medizinischen oder dentalmedizinischen Instruments so auszugestalten, daß eine bessere oder einfache Funktion oder Steuerung eines Pflegegerätes möglich ist.

Diese Aufgabe wird durch die Merkmale des Anspruches 1 gelöst.

Beim System nach Anspruch 1 ist auch das Pflegegerät mit dem Computer verbunden, wobei es auch mit diesem im Datenaustausch steht. Hierdurch läßt sich das Pflegegerät von einem vorhandenen Computer steuern und vereinfachen, da auf eine eigene Steuerung für das Pflegegerät verzichtet werden kann. Darüber hinaus können Steuerdaten ausgenutzt werden, die im Computer des Versorgungsgeräts bereits gespeichert sind, da das Versorgungsgerät auch zur Versorgung des Instruments eingerichtet ist und somit zur Steuerung des Pflegegeräts vorhandene Identifikationsund/oder Betriebsparameter ausgenutzt werden können.

Es ist besonders vorteilhaft, den Istwert des wenigsten einen Betriebsparameters oder -merkmals zu ermitteln und zu registrieren. Dies ist u. a. zur Überwachung und/oder zur Verbesserung der Sicherheit vorteilhaft, weil nicht z. B. ein werksseitig oder benutzerspezifisch bestimmter Wert, z.B. eine Werkzeugdrehzahl, bei einer Behandlungsvorrichtung oder eine Sterilisationstemperatur bei einem Sterilisator, sondern der tatsächlich erreichte Istwert ermittelt wird und registrierbar ist. Es wird somit der Parameter-Wert berücksichtigt, der aus sicherheitstechnischen Gründen bezüglich einer Benutzungsmaßnahme und/oder Pflegemaßnahme wichtig ist. Dies wird z. B. bei einer Pflegevorrichtung in Form eines Sterilisators deutlich. Wenn der Sterilisator den bei der Pflegephase vorgegebenen Istwert der Sterilisationstemperatur nicht erreicht, kann dies mit dem erfindungsgemäßen System ermittelt bzw. festgestellt und somit nachvollzogen und ggf. auch dokumentiert werden. Dies ist aus Gründen der Erbringung eines Nachweises besonders wichtig, z. B. hinsichtlich Garantieansprüchen und/oder Gewährleistung.

Das Registrierungsmittel kann wenigstens einem Gerät und/oder dem Instrument zugeordnet und durch einen elektronischen Speicher oder durch einen Speicherchip gebildet sein, dem der oder die Sollwerte und/oder Istwerte des bzw. der Parameter oder anderer benutzungsspezifischer und/oder pflegespezifischer Informationen in Form von Daten übermittelt und gespeichert, z. B. während der Benutzungsphase und/oder Pflegephase gespeichert werden können.

Die Erfindung ermöglicht es auch, die Sicherheit dahingehend zu Verbessern, dass eine falsche Benutzung der Maßnahmen und/oder eine falsche Pflegemaßnahme vermieden wird. Hierzu kann ein mit dem Registrierungsmittel verbundenes Steuermittel so ausgebildet sein, dass eine Benutzung eines nicht oder unzureichend vorbereiteten, insbesondere unzureichend sterilisierten, Instruments durch Fehlermeldung oder Sperrung der Funktion oder dergleichen vermieden wird. In entsprechender Weise ist es auch möglich, eine Pflegemaßnahme zu vermeiden, z. B. anzuzeigen oder zu sperren, für die das Instrument nicht geeignet ist, z.B. ein Sterilisationsverbot.

Bei der Erfindung kann die Übermittlung der Daten zwischen den Speicher- und Steuermitteln wenigstens eines Gerätes und wenigstens einem Instrument durch leitungslose Fernsteuerung (leitungslose Übertragungsstrecke, z. B. Funk- oder Infrarot-Übertragungsstrecke) oder mittels elektrischen oder optischen Leitungen übermittelt werden.

In Unteransprüchen sind Merkmale enthalten, die die Ausgestaltung und Funktion der Datenleitung im Bereich einer Kupplung für das Instrument verbessern und zu kleinen Bauweisen führen.

Nachfolgend werden vorteilhafte Ausgestaltungen der Erfindung anhand von bevorzugten Ausführungsbeispielen und Zeichnungen näher erläutert. Es zeigen

Fig. 1 ein medizinisches oder dentalmedizinisches Benutzungsgerät in schematischer Seitenansicht;

Fig. 2 ein medizinisches oder dentalmedizinisches Instrument als Teil einer Mehrfachausrüstung für das Benutzungsgerät;

Fig. 3 ein erstes Pflegegerät in Form eines Sterilisators in schematischer Seitenansicht;

Fig. 4 das erste Pflegegerät in Form eines Sterilisators in abgewandelter Ausgestaltung in schematischer Seitenansicht;

Fig. 5 ein zweites Pflegegerät in Form eines Reinigungs- und/oder Schmiergerätes in schematischer Seitenansicht;

Fig. 6 ein Ausführungsbeispiel für die in Fig. 1 mit X gekennzeichnete Einzelheit in vergrößerter Darstellung;

Fig. 7 die Einzelheit X in abgewandelter Ausgestaltung;

Fig. 8 die Einzelheit X in weiter abgewandelter Ausgestaltung;

Fig. 9 den Schnitt IX-IX in Fig. 8;

Fig. 10 die Einzelheit X in weiter abgewandelter Ausgestaltung;

Fig. 11 die Einzelheit X in abgewandelter Ausgestaltung in der Seitenansicht;

Fig. 12 ein Instrument mit der Einzelheit X in weiter abgewandelter Ausgestaltung;

Fig. 13      den Schnitt XIII-XIII in Fig. 12;

Fig. 14a 12      und 14b den zeitlichen Verlauf der in dem Ausführungsbeispiel nach den Fig. und 13 erzeugten Spannungen;

Fig. 15      die Einzelheit X in weiter abgewandelter Ausgestaltung;

Fig. 16      die Einzelheit X in weiter abgewandelter Ausgestaltung.

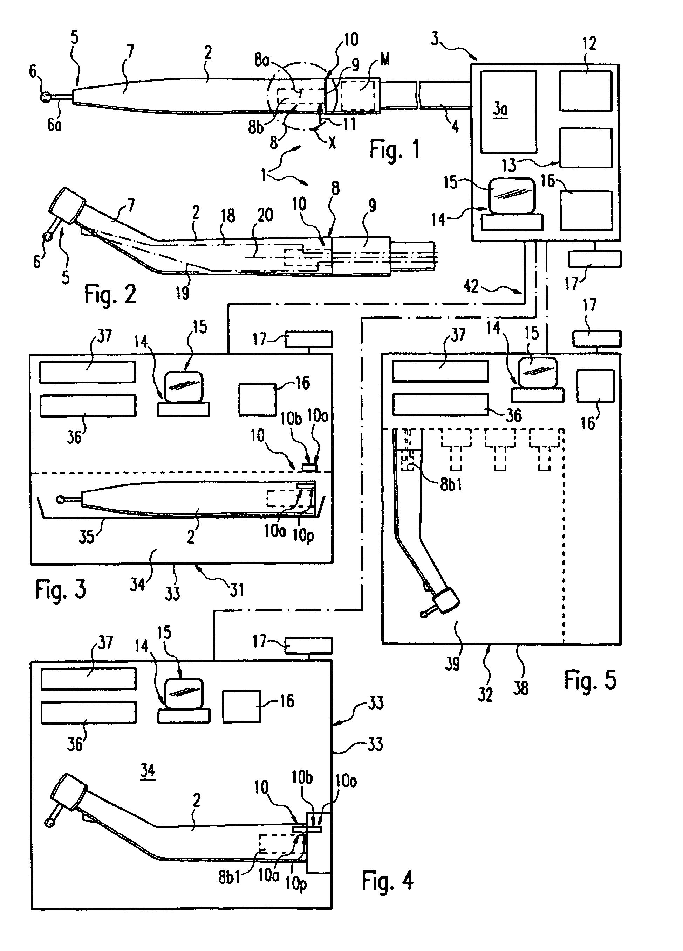

Die Hauptteile der in ihrer Gesamtheit mit 1 bezeichneten Benutzungsvorrichtung bzw. Benutzungsgerätes sind ein Instrument 2 zur Bearbeitung des menschlichen oder tierischen Körpers oder Teilen (Prothesen) davon, ein Versorgungsgerät 3 zum Versorgen des Instruments 2 mit Energie sowie Antriebs- und Benutzungsmedien wie elektrischer Strom, Licht, Wasser und Luft, eine biegsame bzw. flexible Versorgungsleitung 4, die an ihrem hinteren Ende mit dem Versorgungsgerät 3 verbunden ist und an ihrem vorderen freien Ende durch eine lösbare Kupplung mit dem Instrument 2 verbunden ist. Beim Ausführungsbeispiel nach Fig. 1 ist das Instrument 2 ein spanabhebendes Instrument 2, z. B. ein Bohrinstrument, das in seinem vorderen Bereich eine Halte- bzw. Spannvorrichtung 5 aufweist, in die ein Werkzeug 6 mit einem Werkzeugschaft 6a lösbar eingesteckt ist. Das Instrument 2 ist von länglicher bzw. stabförmiger Bauform und erstreckt sich gemäß Fig. 1 beispielsweise gerade. Bei den vorliegenden Ausführungsbeispielen ist das Instrument ein sogenanntes Handstück 7, das durch eine lösbare Kupplung 8, insbesondere eine Steckkupplung oder eine Steck/Drehkupplung, mit einem sogenannten Anschlußteil 9 verbunden ist, das unlösbar oder lösbar, z. B. durch eine Schraubverbindung, mit der Versorgungsleitung 4 verbunden ist. Die Kupplung 8 ist vorzugsweise durch eine Kupplungsausnehmung 8a in dem einen Kupplungsteil und einen mit Bewegungsspiel in die Kupplungsausnehmung 8 passenden Kupplungszapfen 8b am anderen Kupplungsteil gebildet. Vorzugsweise ist die Kupplungsausnehmung 8a im hinteren Endbereich des Handstücks koaxial angeordnet, wobei sie nach hinten ausmündet, und der Kupplungszapfen 8b ragt vom Anschlußteil 9 nach vorne. Die Kupplungsausnehmung 8a und der Kupplungszapfen 8b sind vorzugsweise rund ausgebildet, so daß eine relative Drehbarkeit zwischen diesen Teilen gewährleistet ist, die die Handhabung des Instruments 2 bzw. des Handstücks 7 erleichtert. Der Steckkupplung bzw. Steck/Drehkupplung 8 ist eine lösbare Verrastungsvorrichtung 11 zugeordnet, die vorzugsweise manuell überdrückbar ist und somit beim Zusammenstecken selbsttätig in Funktion tritt und durch ein Überdrücken durch eine manuelle Zugausübung außer Funktion bringbar ist. Eine solche Verrastungsvorrichtung 11 ist vorzugsweise durch eine Verrastungskante an dem einen Kupplungsteil und ein Verrastungselement an dem anderen Kupplungsteil gebildet, das gegen eine elastische Rückstellkraft aus einer die Verrastungskante hintergreifenden Kupplungsstellung bewegbar ist. Hierdurch schnappt die Verrastungsvorrichtung 11 beim Zusammenstecken der Kupplungsteile selbsttätig ein, und sie ist zum Lösen wie vorbeschrieben manuell überdrückbar.

Das Versorgungsgerät 3 weist zu seiner Steuerung und Funktion ein Schalttableau 12 zum Einstellen gewünschter Funktion, eine elektronische Steuereinrichtung 13 mit einer Auswerteschaltung zum Auslesen der gelesenen Daten und zum Steuern und Regeln der Funktionen auf, wobei ein Computer 14 zur Steuerung bzw. Regelung integriert sein kann, insbesondere ein PC mit einem Bildschirm 15 zur Anzeige von Betriebsparametern und -daten des Instruments 2 bzw. Werkzeug 6 und des Versorgungsgeräts 3 sowie zugehöriger Funktionen und Zustände, und eine Registriervorrichtung 16 sowie einen Drucker 17 zur Registrierung bzw. Aufzeichnung der vorgenannten Daten oder Informationen.

Zur Ausübung unterschiedlicher Bearbeitungen können der Benutzungsvorrichtung 1 mehrere Instrumente 2 zugeordnet sein, wie es an sich bekannt ist. Fig. 2 zeigt beispielhaft ein Instrument 2 in Form eines sogenannten Winkelstücks. Bei dem oder den Instrumenten 1 kann es sich somit um solche unterschiedlicher Bauweise und Funktion handeln, die bezüglich der Kupplung 8 jeweils passend ausgebildet sind, so daß sie wahlweise an das Anschlußteil 9 ankuppelbar und austauschbar sind. Es kann sich um ein Instrument mit einer sich daran axial erstreckenden Antriebswelle handeln, die durch einen im Anschlußteil 9 angeordneten, z. B. elektrischen Motor M drehbar ist und mit dem Werkzeug 6 in Antriebsverbindung steht. Das Werkzeug 6 kann auch durch eine im vorderen Endbereich des Instruments 2 angeordnete Druckluftturbine antreibbar sein, die durch eine sich vom Versorgungsgerät 3 und längs durch die Versorgungsleitung 4, das Anschlußteil 9 und das Instrument 2 erstreckende Druckluftzuführungsleitung und -abführungsleitung versorgt wird. Zur Zuführung von Benutzungsmitteln bzw. Bearbeitungsmitteln kann das Instrument eine Luftleitung, eine Wasserleitung oder eine Sprayleitung aufweisen, die in an sich bekannter Weise im vorderen Endbereich des Instruments 1 austreten und auf die Bearbeitungsstelle gerichtet sind. Das Instrument 2 kann auch eine Beleuchtungsvorrichtung mit einem sich längs erstreckenden Lichtleiter oder einer sich längs erstreckenden elektrischen Leitung mit Lampe aufweisen, die sich zu einem im vorderen Endbereich des Instruments 1 angeordneten Lichtaustritt erstrecken. Zur Versorgung der vorbeschriebenen Einrichtungen dienen sogenannte Medienleitungen, die sich von einer Versorgungseinheit 3a des Versorgungsgeräts 3 durch die Versorgungsleitung 4 zum zugehörigen Verbraucher im Anschlußteil 9 oder im Instrument 2 erstrecken. Wenn eine Dreh/Steckkupplung vorgesehen ist, kann eine Medienleitung diese insbesondere koaxial abgedichtet durchsetzen und/oder es können mehrere Medienleitungen die zylindrische Teilungsfuge zwischen dem Kupplungszapfen 8a und der Kupplungsausnehmung 8b Z-ftirmig abgedichtet durchsetzen, wie es an sich bekannt ist. Zur Verdeutlichung dieser an sich bekannten Sachverhalte sind in Fig. 1 beispielhaft drei Medienleitungen 18, 19, 20 für Wasser, Druckluft und Licht andeutungsweise dargestellt, die die Kupplung 8 koaxial bzw. Z-förmig durchsetzen.

Zur Einstellung unterschiedlicher Benutzungs- bzw. Betriebsprogramme weist die Benutzungsvorrichtung 1 wenigstens zwei Programmebenen auf. In einer ersten Programmebene kann der Benutzer zwischen einer Vielzahl von hersteller- bzw. werkseitig vorgegebenen Behandlungs- bzw. Betriebsprogrammen auswählen. In einer zweiten Programmebene kann der Benutzer zwischen einer Vielzahl von benutzerspezifisch erstellten Betriebsprogrammen auswählen. Nach Auswahl eines der Betriebsprogramme steuert die Steuereinrichtung 13 das Instrument 2 bzw. das Werkzeug 6 abhängig von den dem ausgewählten Betriebsprogramm entsprechenden Betriebsparameterinformationen bzw. -daten. Der Benutzer kann beliebig zwischen den benutzerspezifisch abgespeicherten Betriebsprogrammen und den herstellerseitig vorgegebenen Betriebsprogrammen wählen.

Die jedem Betriebsprogramm zugeordneten Betriebsparameterdaten enthalten Informationen über ein auszuwählendes Instrument 2 und dessen betriebs- und/oder benutzungsspeziflsche Daten, z. B. Drehzahl, Untersetzungsverhältnis, Wirkungsgrad, Benutzungsmittel sowie Benutzungsmittelmenge usw.

Die Registriervorrichtung 16 ermöglicht es, gewünschte betriebs- und benutzerspezifische Daten und Informationen über die jeweilige Betriebszeit hinaus zu registrieren und abzurufen, z. B. zwecks Anzeige am Bildschirm 15 oder durch Ausdruck mit dem Drucker 17.

Zur Identifizierung der Instrumente 2 weisen diese im Bereich des Kupplungsteils des Instruments 2 ein Codierung 10a auf, die von einer Lesevorrichtung 10b lesbar ist, die im Bereich des anderen Kupplungsteils am Anschlußteil 9 bzw. an der Versorgungsleitung 4 angeordnet ist und durch wenigstens eine zugehörige Datenleitung mit der Steuereinrichtung 13 verbinden ist.

Die Registriervorrichtung 16 ist vorzugsweise dazu eingerichtet, bei solchen Daten, die im Funktionsbetrieb variabel sein können, nicht nur den z. B. vom Benutzer eingestellten Sollwert, sondern auch den tatsächlich erreichten Istwert zu registrieren. Hierbei kann es sich z. B. um eine Maximaldrehzahl handeln, die je nach Belastung und Leistung unterschiedlich sein kann. Die Registrierung des Istwertes ermöglicht es, den tatsächlichen Sachverhalt festzuhalten, abzurufen und darzustellen, um z. B. die Benutzung auch nachträglich verdeutlichen und beurteilen zu können.

Die mit 10 bezeichneten Identifikationsvorrichtung kann z. B. optisch, elektromagnetisch oder induktiv wirksam sein. Bei einer Steck/Drehkupplung 8 mit einer Kupplungsausnehmung 8a und einem Kupplungszapfen 8b ist es vorteilhaft, die Identifikationsvorrichtung zu beiden Seiten der zylindrischen oder radialen Teilungsfuge der Kupplung 8 anzuordnen. Die Codierung 10a kann beim Ausführungsbeispiel in oder an der die Kupplungsausnehmung 8a umgebenden Hülsenwand 2a des Instruments 2 angeordnet sein, wobei die Lesevorrichtung 10b im Kupplungszapfen 8b oder der Hülsenwand 2a gegenüberliegend im Anschlußteil 9 angeordnet sein kann. Im ersteren Fall ist die Wirkrichtung der Identifikationsvorrichtung 10 radial, wobei die Daten der Lesevorrichtung 10b von der Mantelfläche des Kupplungszapfens 8b her zugänglich sind, während im zweiten Fall die Lesevorrichtung 10b im axialen Projektionsbereich der Hülsenwand 2a angeordnet und mit den Daten von der Ringstufenfläche am Anschlußteil 9 her zugänglich ist.

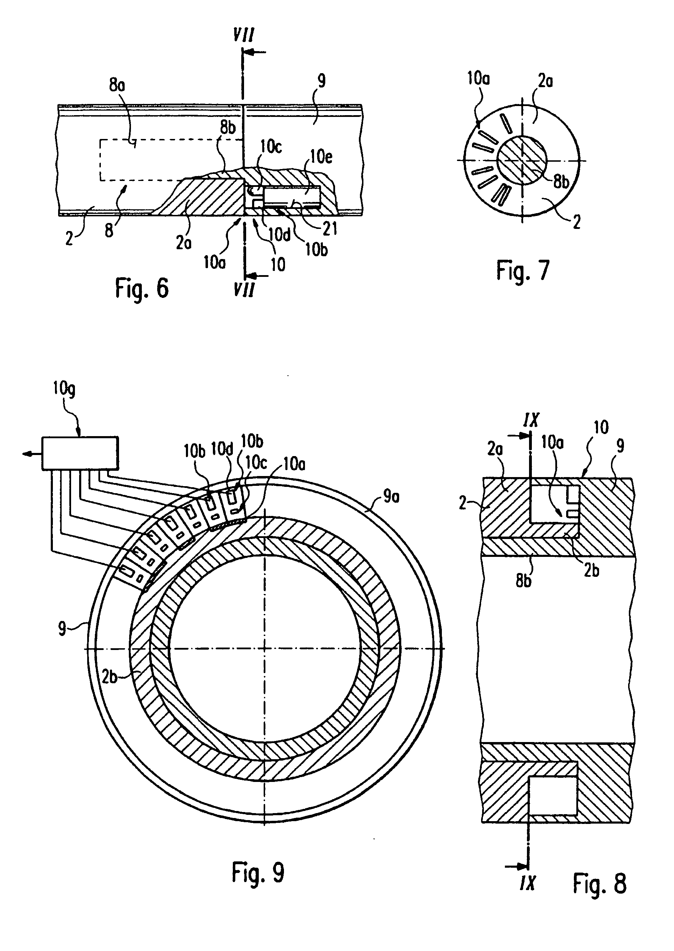

Beim Ausführungsbeispiel nach Fig. 6, bei dem gleiche oder vergleichbare Teile mit gleichen Bezugszeichen versehen sind, ist eine optische Identifikationsvorrichtung 10 mit einer Codierung 10a, z. B. in Form eines Mustercodes, insbesondere eines Strichcodes, an einer Rückenfläche, hier die Rückenfläche der Hülsenwand 2a, und einer Lesevorrichtung 10b hinter der radialen Teilungsfuge im Anschlußteil 9. Diese Lesevorrichtung 10b weist eine z. B. durch eine Leuchtdiode gebildete Lichtquelle 10c und einen Photozellen-Sensor 10d auf, die vorzugsweise - in einem gemeinsamen, insbesondere zylindrischen Träger 10e angeordnet bzw. eingebettet sind, der in einer Ausnehmung 21 fest eingesetzt ist, die an der Ringstufe der radialen Teilungsfläche mündet. Der Mustercode (Hell- und Dunkelflächen) ist vorzugsweise durch radiale Striche gebildet, zwischen denen unterschiedliche Winkel eingeschlossen sind, wie es Fig. 7 zeigt. Die Leserichtung verläuft axial bzw. achsparallel.

Beim Ausführungsbeispiel nach Fig. 8 und 9, bei dem gleiche oder vergleichbare Teile mit gleichen Bezugszeichen versehen sind, ist Die Leserichtung der Lesevorrichtung 10b radial gerichtet, hier radial nach innen. Beim Ausführungsbeispiel sind mehrere Photozellensensoren 10d bezüglich der Codierung 10a in Umfangsrichtung nebeneinander und radial außen angeordnet. Die Codierung 10a kann durch eine Hell-Dunkelmarkierung auf einer äußeren Umfangs- oder Segmentfläche gebildet sein, insbesondere durch einen Strichcode. Wie Fig. 9 zeigt, sind die Codierung 10a und die Lesevorrichtung 10b an einander radial gegenüberliegenden Teilen der Kupplungsteile angeordnet. Hierbei kann die Codierung 10a an der Außenmantelfläche eines inneren Rücksprunges 2b am Instrument 2 angeordnet sein, der von der Lesevorrichtung 10b übergriffen ist. Die vorzugsweise in einer Mehrzahl vorhandenen Photozellen können zum einen an einer nach vorne gerichteten Stirnfläche des Anschlußteils 9 angeordnet und zum anderen in ein Sensorringsegment zusammengefaßt sein. Ein die Lesevorrichtungen 10b aufnehmender Freiraum kann durch einen vorspringenden Hülsenansatz 9a des Anschlußteils 9 abgedeckt sein. Ein Parallel-Seriell-Umsetzer oder eine Auswerteschaltung ist mit 10g bezeichnet.

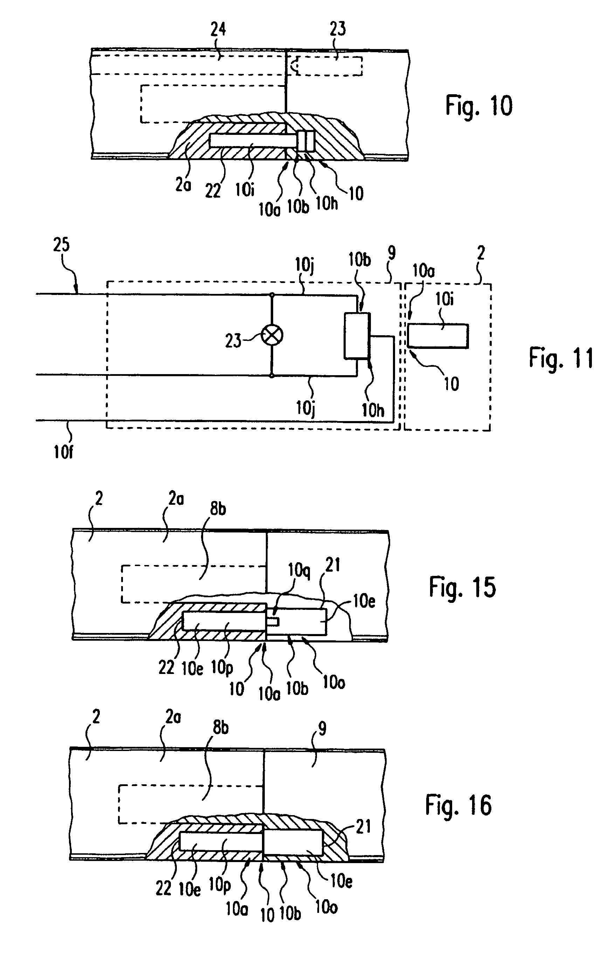

Das Ausführungsbeispiel nach Fig. 10 und 11 zeigt eine Identifikationsvorrichtung 10 mit einem insbesondere magnetischen Sensor 10h, z. B. ein Hallsensor oder eine Feldplatte, in entsprechender Anordnung hinter der radialen Teilungsfuge des Anschlußteils 9. Dem Sensor 10h ist in der Hülsenwand 2a ein Magnet 10i gegenüberliegend in einer rückseitig offenen Ausnehmung 22 angeordnet ist. Fig. 11 zeigt schematisch die Anordnung und den elektrischen Anschluß des Sensors 10h beim Vorhandensein einer Beleuchtungseinrichtung mit einer Lampe 23, die beim Ausführungsbeispiel bezüglich des Sensors 10h in Umfangsrichtung versetzt im Anschlußteil 9 angeordnet ist und Licht in einen der Lampe 23 gegenüberliegend im Instrument 2 angeordneten und sich in der Hülsenwand 2a nach vorne erstreckenden Lichtleiter 24 einspeist. Der Sensor 10h ist durch elektrische Leitungsabschnitte 10j an den Stromkreislauf 25 der Lampe 23 angeschlossen. Die Codierung 10a kann bei diesem Ausführungsbeispiel dadurch gebildet sein, daß unterschiedliche Instrumente 2 Magnete 10i mit unterschiedlich starken elektromagnetischen Feldern aufweisen und/oder die Luftspaltabstände zwischen dem Hallsensor 10h und den Magneten 10i jeweils im gekuppelten Zustand unterschiedlich sind. Hierdurch werden im Sensor 10h unterschiedliche elektrische Spannungen erzeugt, die durch eine sich vom Sensor 10h durch das Anschlußteil 9 und die Versorgungsleitung 4 nach hinten zur elektronischen Steuereinrichtung 13 erstreckenden Signalleitung der elektronischen Steuereinrichtung 13 zur Einstellung der gewünschten Betriebsdaten zugeführt werden.

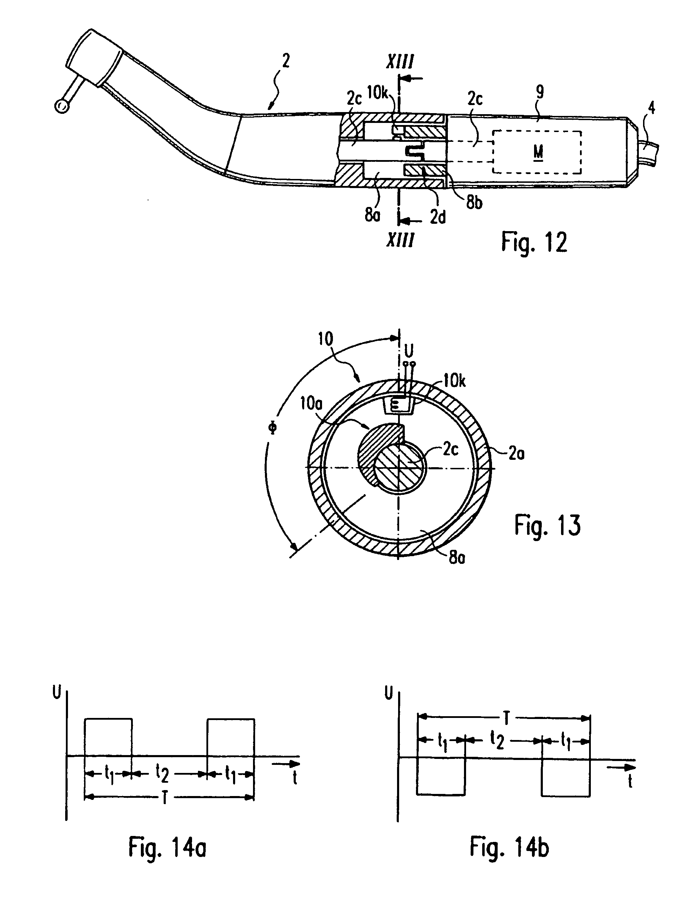

Beim Aushührungsbeispiel nach Fig. 12 bis 14 ist die Identifikationsvorrichtung 10 für ein Instrument 2 eingerichtet, in dem im Bereich der Kupplung 8 ein Drehteil gelagert ist, bei dem es sich beim Ausführungsbeispiel um eine Antriebswelle 2c handelt, die in einem Antriebskanal des Instruments 2 drehbar gelagert ist. Das hintere Ende der Antriebswelle 2c ist durch eine Steckkupplung 2d mit einem im Anschlußteil 9 drehbar gelagerten Antricbswellenabschnitt 9a gekuppelt, bei dem es sich z.B. um die Antriebswelle des Motors M handeln kann. Bei dieser Ausgestaltung ist ein Sensor 10k vorgesehen, der am Anschlußteil 9 gehalten ist, und zwar an der Stirnseite des Kupplungszapfens 8b. Auf der Antriebswelle 2c des Instrumentes 2 befindet sich ein Geberelement 10a aus ferromagnetischem Material, das sich über einen Winkel Φ erstreckt und eine über den Winkel Φ abnehmende Höhe hat. Es hat - in der Abwicklung gesehen - die Form einer Rampe. Das Geberelement 10a passiert bei Drehung der Antriebswelle 2c den Sensor 10k. Der Sensor 10k ist z.B. eine Spule, in der das sich vorbeibewegende Geberelement 10a eine von der Drehgeschwindigkeit und der Drehrichtung der Antriebswelle 2c abhängige Spannung U erzeugt.

Es ist nun eine Codierung der Instrumente 2 durch Wahl der Anfangs- und Endehöhe des Geberelementes 10a und/oder durch Wahl des Winkels Φ, über den sich das Geberelement erstreckt, möglich. Mit anderen Worten, eine Codierung ist durch die Wahl einer bestimmten Rampenform möglich.

In den Fig. 14a und 14b sind die Ausgangssignale des Sensorelementes 10k dargestellt, und zwar als Spannung U in Abhängigkeit von der Zeit. Bei Drehung der Antriebswelle 2c in Fig. 13 entstehen Spannungsimpulse.

In Fig. 14a sind die Spannungsimpulse bei Linksdrehung der Antriebswelle 2c gezeigt. In diesem Fall hat die Rampe in Bezug auf den Sensor 10k eine ansteigende Form, mit der Folge, daß positive Spannungsimpulse erzeugt werden.

In Fig. 14b sind die Spannungsimpulse bei Linksdrehung der Antriebswelle gezeigt. Da die Rampe hier in Bezug auf den Sensor 10k eine abfallende Form hat, sind die Spannungsimpulse negativ.

Die Impulse haben in beiden Figuren eine zeitliche Dauer $t_1$ und einen zeitlichen Abstand $t_2$. Die Periodendauer beträgt T. Der Zusammenhang zwischen dem in Fig. 13 gezeigten Winkel Φ und den in den Fig. 14a und 14b angegebenen Zeiten ergibt sich aus der Formel:

$$\Phi = 360\,° \times t_1/T$$

Die Drehzahl n der Antriebswelle 2c ist n = 1/T. Durch Messung von $t_1$ und $t_2$ bzw. T kann auf den Codierungswinkel Φ geschlossen werden.

Durch Messung der Amplitude der Spannung U kann bei Kenntnis der Drehzahl n auf die Rampenform geschlossen werden, die damit auch zur Codierung herangezogen werden kann.

Die Polarität der Spannungsimpulse gibt Auskunft über den Drehsinn (links oder rechts rum) der Antriebswelle 2c.

Bei den Ausführungsbeispielen nach Fig. 15 und 16 ist jeweils die Codierung 10a veränderlich. Die Codierung 10a ist nicht nur in der Lage, bestimmte Daten bereitzustellen, die von der Lesevorrichtung 10b lesbar sind, sondern die Codierung 10a läßt sich bezüglich der in ihr gespeicherten Daten verändern und zwar mit Daten, die ihr von der elektronischen Steuereinrichtung 13 übertragen werden. Hierbei kann es sich um herstellerseitig vorgegebene oder vom Benutzer erstellte Betriebsdaten, z.B. wenigstens ein Betriebsprogramm, handeln. Es kann sich auch um andere Informationen handeln, z. B. die den Betrieb des Instruments 2 und/oder den zu behandelnden Patienten und/oder die Behandlung des Instruments 2 im Sinne von Pflegemaßnahmen betreffen. Hierbei ist die Lesevorrichtung 10b zugleich Datensender und -empfänger 10o und die Codierung 10a ist durch eine Übertragung von Daten vom Datensender 10o veränderlich und vorzugsweise durch ein Speicherelement 10p zum Überschreiben seiner Daten durch die vom Datensender 10o übertragenen Daten ausgebildet. Fig. 15 und 16 zeigen beispielhaft zwei Ausführungsbeispiele für eine solche Datenübertragungseinrichtung, bei der die Daten wahlweise sowohl in die eine als auch in die andere Richtung zwischen der elektronischen Steuereinrichtung 13 und dem Speicherelement 10p übertragen, d. h. abgerufen und wieder eingelesen werden können.

Fig. 15 zeigt ein Ausführungsbeispiel mit einer mechanischen Kontaktierung 10q zwischen dem Datenempfänger und - sender 10o und dem Speicherelement 10p. Die einzelnen elektrischen Kontakte sind aus Vereinfachungsgründen nicht dargestellt. Fig. 16 zeigt eine berührungslose Datenübertragung zwischen dem Datenempfänger und - sender 10o und dem Speicherelement 10e. Die Datenübertragung kann z.B. elektromagnetisch oder induktiv erfolgen. Das Speicherelement 10p kann durch einen Transponder gebildet sein, nämlich ein Empfangs-Sendegeräte, das nach dem Abfrage-Antwort-System arbeitet. Bei dem Datenempfänger und -sender 10o kann es sich um einen korrespondierenden Transponder handeln. In beiden Fällen handelt es sich um Speicherelemente, die jeweils durch ein vorgefertigtes, insbesondere stiftförmiges Bauteil gebildet und in einer den Ausnehmungen 21,22 entsprechenden Ausnehmung eingesetzt sind.

Aufgrund der Übertragung von solchen Daten auf das Instrument 2, die für eine beabsichtigte Benutzung des Instruments 2 am Patienten und/oder für eine Behandlung des Instruments 2 selbst, z.B. Pflegemaßnahmen, und/oder für eine Registrierung von Daten bestimmend sind, ist es möglich, das Instrument 2 mit einem anderen Gerät zu kombinieren, das einen Datenempfänger bzw. eine Lesevorrichtung aufweist, die diese Daten empfangen und vorzugsweise Daten auf das Speicherelement 10p rücküberträgen kann. Bei einem solchen weiteren Gerät kann es sich z.B. um eine zweite, den Benutzer zur Verfügung stehende Benutzungsvorrichtung (nicht dargestellt) und/oder ein Pflegegerät handeln, was noch beschrieben wird. Dabei bedarf es bei den Ausführungsbeispielen nach Fig. 15 und 16 keiner Datenleitung direkt zwischen dem Versorgungsgerät 3 und dem weiteren Gerät, denn die Datenleitung kann durch das Instrument 2 erfolgen.

Bei allen vorbeschriebenen Aushihrungsbeispielen ist es vorteilhaft, die empfindlichen Bauteile, hier die Lesevorrichtung 10b am Anschlußteil 9 oder am von diesem vorragenden Kupplungszapfen 8b anzuordnen und die Codierung am Instrument 2 anzuordnen. Hierdurch sind die weniger empfindlichen Teile der Identifikationsvorrichtung 10 am Instrument 2 angeordnet, das bei einer Reinigung, Desinfektion und/oder Sterilisation beträchtlichen Beanspruchungen, insbesondere Temperaturen unterliegt. Außerdem ist die elektronische Steuereinrichtung 13 durch wenigstens eine geeignete Datenleitung mit der Lesevorrichtung 10b bzw. mit dem Datenempfänger und -sender 10o verbunden.

Die Fig. 3 bis 5 zeigen jeweils beispielhaft ein solches zweites Gerät als Pflegegerät, wobei Fig. 3 und 4 jeweils ein Sterilisiergerät 31 und Fig. 5 ein Pflegegerät 32 zum Reinigen und Ölen zeigen.

Das Sterilisiergerät 31 nach Fig. 3 weist in einem Gehäuse 33 eine Sterilisierkammer 34 auf, die durch eine nicht dargestellte Tür zu öffnen und zu schließen ist, so daß ein zu sterilisierendes Instrument 2 in die Sterilisierkammer 34 eingebracht und abgelegt werden kann, z.B. auf eine Ablage oder Schale 35. Um mit Instrumenten 2 gemäß den vorbeschriebenen Ausgestaltungen nach Fig. 6 bis 11 und Fig. 15 und 16 benutzt werden zu können, ist dem Sterilisiergerät 31 eine Lesevorrichtung 10b oder ein Datenempfänger und -sender 10o zugeordnet, der sich in oder außerhalb der Sterilisierkammer 34 befinden kann und das Instrument 2 anhand der Codierung 10a identifizieren kann und die für die Desinfizierung erforderlichen Versorgungsmaßnahmen durchführt, wozu z.B. das Erwärmen der Sterilisierkammer 34 auf eine erforderliche Sterilisierungstemperatur, z.B wenigstens 135°, und ggf. auch das Erzeugen eines Über- oder Unterdrucks in der Sterilisierkammer 34 umfassen. Mit den Bezugszahlen 36 und 37 sind andeutungsweise ein Versorgungsgerät zum Bereitstellen der Sterilisiermedien und eine elektronische Steuereinrichtung mit einer Auswerteschaltung für die gelesenen Daten und zum Steuern der Sterilisiermaßnahmen zugeordnet. Die von der Lesevorrichtung 10b bzw. vom Datenempfänger 10o gelesenen Daten werden der Steuereinrichtung 37 zwecks Erkennung des Instruments 2 und Steuerung der Sterilisiermaßnahmen zugeführt.

Das Ausführungsbeispiel gemäß Fig. 4 unterscheidet sich von dem gemäß Fig. 3 dadurch, daß in der Sterilisierkammer 34 ein dem Kupplungszapfen 8b nach Form und Größe entsprechender Kupplungszapfen 8b1 angeordnet ist, z.B. an einer Wand der Sterilisierkammer 34 befestigt ist, auf den das Instrument 2 mit seiner Kupplungsausnehmung 8a aufsteckbar ist. Auch bei diesem Ausfuhrungsbeispiel kann die Kupplung 8 entsprechend umgekehrt ausgebildet sein, d.h. es kann im Sterilisierraum 34 eine Kupplungsausnehmung angeordnet sein, in die ein Instrument 2 mit einem abstehenden Kupplungszapfen einsteckbar ist. Hierdurch ist in der Sterilisierkammer 34 eine Positioniervorrichtung für das Instrument 2 geschaffen, die die Positionierung und Erkennung des Instruments 2 und der Daten erleichtert.

Das Pflegegerät 32 gemäß Fig. 5 weist in einem Gehäuse 38 eine Pflegekammer 39 auf, die durch eine nicht dargestellte Tür wahlweise zu öffnen und zu schließen ist, und in die für einen Pflegevorgang das Instrument 2 in die Pflegekammer 39 eingebracht und positioniert wird, z. B. auf eine Ablage oder Schale oder an einem Kupplungsteil, insbesondere an einem Kupplungszapfen 8b1, wie es für das Sterilisiergerät 31 bereits beschrieben worden ist. Das Pflegegerät 32 kann für unterschiedliche Instrumente 2 mehrere Kupplungsteile bzw. Kupplungszapfen 8b1 aufweisen, wie es in Fig. 5 andeutungsweise dargestellt ist. Auch die Sterilisiergeräte 31 gemäß Fig. 3 und 4 können mehrere solcher Kupplungsteile für unterschiedliche Instrumente 2 aufweisen. Auch das Pflegegerät 32 weist ein Versorgungsgerät 36 und eine elektronische Steuereinrichtung 37 mit einer Auswerteschaltung für die gelesenen Daten zum Bereitstellen von Pflegemedien, z.B. Druckluft oder ein Pflegemittel, insbesondere Öl, und Zuführen dieser Medien zum Instrument 2 und zum Steuern dieser Maßnahmen zwecks Durchführung der Reinigung und/oder Pflege. Das Pflegegerät 32 kann im Übrigen von der eingangs beschriebenen Art sein.

Wie bereits das Pflegegerät 13 weist auch das Pflegegerät 32 jeweils eine den zugehörigen Kupplungsteilen 8b1 zugeordnete Lesevorrichtung 10b oder einen Datenempfänger und -sender 10o auf, die bzw. der das eingebrachte bzw. angekuppelte Instrument 2 anhand einer Codierung 10a bzw. Datenspeicherung erkennt und die Daten an die Steuereinrichtung 37 zur Steuerung des Versorgungsgeräts 36 und zur Ausführung der Pflegemaßnahmen weiterleitet.

Im ersten oder zweiten Pflegegerät, hier dem Sterilisiergerät 31 und dem Pflegegerät 32 oder einem zusätzlichen oder anderen Gerät kann jeweils ein Computer 14a gegebenenfalls mit Bildschirm 15 zur Verarbeitung der jeweiligen Daten und zur Steuerung entsprechend den Daten zugeordnet sein. Außerdem kann jedem dieser Geräte eine Registriervorrichtung 16 zur Registrierung von Betriebsdaten und Informationen z. B. zur Registrierung der durchgeführten Pflegemaßnahmen, insbesondere der Sollwerte und Istwerte dieser Betriebsdaten zugeordnet sein. Hierbei kann es sich bei dem Sterilisiergerät 31 z.B. um die Sterilisierzeit und/oder die Sterilisiertemperatur und/oder den Sterilisierüberdruck oder - unterdruck handeln. Beim Pflegegerät 32 kann es sich z.B. um die Pflege- bzw. Funktionszeit von Pflegemaßnahmen wie Durchblaszeit und/oder Ölungszeit und/oder Ölmenge handeln.

Beim Ausführungsbeispiel sind vereinfacht dargestellte Zuführungsleitungen 41 für die Pflegernedien, hier Druckluft und Öl, vorhanden, die sich von einem jeweils zugehörigen Vorrat zu dem oder den Kupplungsteilen 8b1 erstrecken und im angekuppelten Zustand des Instruments 2 mit den Medienkanälen des Instruments 2, hier den Kanälen 18, 19 und/oder dem einen sog. Antriebskanal, in dem die Antriebswelle des Instruments 2 drehbar gelagert ist, in Verbindung steht zwecks Reinigung durch ein Durchblasen von Druckluft und Pflege des Antriebskanals durch Einführung bzw. Einblasung von Pflegemittel, insbesondere Öl.

Bei einer Ausgestaltung gemäß Fig. 15 und 16 kann auf eine direkte Vernetzung der Geräte 1 und/oder 31 und/oder 32 durch Datenleitungen 42 verzichtet werden, da die erforderlichen Daten im Instrument 2 gespeichert werden können und mit dem Instrument 2 an das jeweilige Gerät übertragen bzw. vom jeweiligen Gerät abgerufen werden können. Bei den Ausgestaltungen, bei denen die Codierung 10a mittels der Lesevorrichtung 10b nur gelesen werden kann, ist eine Vernetzung 42 der wenigstens zwei Geräte erforderlich. Die Vernetzung kann mittels besonderer elektrischer Leitungen oder kabellos erfolgen, beispielsweise über Infrarot- oder Funkübertragungsstrecken. Es ist möglich, den wenigstens zwei Geräten 1 und/oder 31 und/oder 32 und/oder wenigstens einem weiteren Gerät, z. B. ein zweites Benutzungsgerät, mit einer gemeinsamen elektronischen Steuereinrichtung 13 bzw. mit einem gemeinsamen Computer 14, einer gemeinsamen Registriervorrichtung 16 und einem gemeinsamen Drucker 17 auszurüsten. Bei einer solchen Ausgestaltung ist eine Vernetzung 42 auch dann erforderlich, wenn Instrumente 2 gemäß Fig. 15 und 16 zu pflegen bzw. zu behandeln sind, da die Steuerung gemeinsame erfolgt.

Nachfolgend werden diverse Vorteile und Funktionen der erfindungsgemäßen Ausgestaltungen beschrieben.

Beim Ausführungsbeispiel nach Fig. 1 und 2 bildet das Benutzungsgerät 1 mit einem gegebenenfalls vorhandenen Behandlungsstuhl einen Hehandlungsplatz, wobei es sich um ein Chirurgiegerät, insbesondere ein Mikrochirurgiegerät, oder um ein dentales Behandlungsgerät handeln kann. Aufgrund der Registrierung nicht nur der Sollwerte, sondern auch der Istwerte von Betriebsdaten, z. B. bei der Benutzung aufgetretene maximale Drehmomente oder maximale Werkzeugdrehzahlen, und weitere Betriebsinformationen, wie z. B. die Uhrzeit oder das Datum, lassen sich nicht nur während der Bearbeitung sondern auch nach der Bearbeitung abrufen und vorübergehend oder bleibend darstellen, z. B. am Bildschirm 15 und durch Ausdrucken am Drucker 17. Es kann somit die Benutzung des Instruments 2 bei der Bearbeitung des Patienten oder Körpers und/oder die Behandlung des Instruments 2 selbst, z. B. zum Zweck der Pflege, nachgewiesen und beurteilt werden, was insbesondere im Hinblick auf Garantieansprüche und Lebensdauer von Bedeutung ist.

Diese Vorteile und Maßnahmen gelten nicht nur für ein Behandlungsgerät nach Fig. 1 und 2 sondern auch für ein Pflegegerät 32 zur Durchführung von Reinigungs- und/oder Desinfizier- und/oder Sterilisiermaßnahmen.

Die erfindungsgemäßen Ausgestaltungen ermöglichen das Erkennen und Erfüllen folgender Anforderungen des bzw. der Instrumente 2.

a) Um welches Instrument 2 handelt es sich?
b) Welche Anforderungen stellt das Instrument 2 ? Z. B. Betriebsdaten wie Drehzahl, Drehmoment, welches Benutzungsmedium (Luft, Wasser, elektrischer Strom, usw. ist erforderlich?
c) In welchem Zustand befindet sich das Instrument ? Z. B. wieviele Betriebsstunden hat das Instrument bereits gelaufen, ist das Instrument 2 gereinigt oder desinfiziert oder sterilisiert oder gepflegt?

Die Anforderungen a) und b) erkennt die jeweils einzelne oder gemeinsame elektronische Steuereinrichtung durch Vergleich und Auswertung der erkannten Identifikationsdaten mit in der elektronischen Steuereinrichtung gespeicherten Daten. Die Anforderung c) wird dadurch erfüllt, daß die betreffenden Betriebsdaten ermittelt und registriert werden und somit die betreffenden Registraturdaten den jeweiligen Zustand des Instruments 2 repräsentieren. Durch eine fortlaufende Registrierung von Daten läßt sich auch der Betriebsablauf registrieren und somit nicht nur bestimmte Betriebsfunktionen der Benutzung und/oder der Pflege registrieren. Es lassen sich auch bestimmte Betriebsabläufe oder Daten so in Abhängigkeit von vorherigen Betriebsabläufen oder gespeicherten Daten steuern, daß ein bestimmter Betriebsablauf nur dann erfolgt, wenn ein vorheriger Betriebsablauf stattgefunden hat oder ein bestimmter Betriebszustand erreicht wird, der durch Daten registriert oder gespeichert ist. Wenn z. B. ein Instrument 2 nicht gepflegt worden ist, erkennt die Steuereinrichtung diesen Mangel aufgrund des Fehlens betreffender Daten, und sie sperrt den nachfolgenden Betriebsablauf oder zeigt diesen Mangel durch ein Warnsignal an oder registriert diesen Mangel. Wenn z. B. ein Instrument 2 nicht gepflegt worden ist oder unzureichend gepflegt worden ist, wenn z. B. die Sterilisiertemperatur und/oder die Sterilisierzeit nicht dem Sollwert entspricht, erkennt dies die Steuereinrichtung spätestens beim Ankoppeln des Instruments an das Versorgungsgerät 3, wobei die Steuereinrichtung die Benutzung sperren kann oder zumindest den Mangel anzeigen kann.

Eine solche Sperrung oder Anzeige läßt sich auch dann steuern, wenn ein Instrument 2 eine vorgegebene Anzahl Betriebsstunden überschreitet, die ebenfalls durch Registrierung zugehöriger Daten überwacht werden können.

Der Erfindung liegt die Erkenntnis zugrunde, daß Betriebsabläufe und Zustände von wenigstens einem Instrument 2 an wenigstens zwei Benutzungs- und/ oder Versorgungsgeräten erkannt werden können, wenn die Geräte und/oder die Instrumente mit entsprechenden Erkennungsdaten gespeichert sind. Ein Instrument 2 wird mit einem Pflegegerät 31 oder 32 zu seiner Pflege gekoppelt. Dieses Gerät erkennt die jeweilige Art des Instruments. Das Gerät ruft nun die gespeicherten Daten für genau dieses Instrument 2 ab. Nach diesen Vorgaben (Ölmenge, Ausblaszeit) startet der Pflegezyklus. Gespeichert werden nach abgeschlossener Pflege zugehörige Durchlauf- oder Pflegedaten, wozu auch die Seriennummer des Instruments 2 dienen kann.

Wenn auch wenigstens ein anderes Gerät in der Lage ist, die Daten des vorherigen Betriebsablaufs abzurufen und zu speichern, kann man durch die Datenvernetzung der Geräte Betriebsabläufe mehrerer Geräte nachverfolgen, z. B. die Pflege und die Benutzung des Instruments 2.

Z. B. Instrument A wurde am 16.01.2001 um 10.00 Uhr im Pflegegerät 32 gepflegt. Instrument A wurde am 16.01.2001 um 10.05 Uhr im Sterilisiergerät 31 sterilisiert. Instrument A wurde am 16.01.2001 um 11.00 Uhr am Benutzungsgerät 1 (Patient 4) insgesamt fünf Minuten betrieben. Dadurch kann nicht nur der Betriebsablauf festgehalten werden, sondern es können auch Fehler festgestellt werden, z. B. durch eine Fehlermeldung, die die zugehörige Steuereinrichtung errechnet oder beim Koppeln des nicht sterilisierten Instruments 2 erkennt.

## Patentansprüche

1. System zum Benutzen und Pflegen eines medizinischen oder dentalmedizinischen Instruments (2), mit einem Versorgungsgerät (3), von welchem das Instrument (2) für die Benutzung mit Medien versorgt wird, und mit einem Pflegegerät (31; 32) zum Pflegen des Instruments (2), wobei das Versorgungsgerät (3) mit einem Computer (14) verbunden ist, der im Datenaustausch mit dem Versorgungsgerät (3) steht,
   und wobei sowohl das Versorgungsgerät (3) als auch das Pflegegerät (31; 32) eine Lesevorrichtung (10b) zum Auslesen von in dem Instrument (2) gespeicherten Identifizierungs- und/oder Betriebsparameterdaten aufweisen,
   **dadurch gekennzeichnet,**
   **dass** auch das Pflegegerät (31; 32) mit dem Computer (14) verbunden ist und mit diesem im Datenaustausch steht
   und **dass** das Pflegegerät ein Kupplungsteil aufweist, durch das das Instrument (2) mit dem Pflegegerät (31; 32) kuppelbar ist, wobei die Lesevorrichtung (10b) im Bereich des Kupplungsteils angeordnet ist.

2. System nach Anspruch 1,
   **dadurch gekennzeichnet,**
   **dass** das Kupplungsteil durch einen vorstehenden Kupplungszapfen (8b1) gebildet ist und die Lesevorrichtung (10b) im Kupplungszapfen (8b1) oder hinter einer Ringstufenfläche, von der der Kupplungszapfen (8b1) vorragt, angeordnet ist.

3. System nach Anspruch 1 oder 2,
   **dadurch gekennzeichnet,**
   **dass** das Instrument (2) Betriebsphasen an wenigstens zwei Geräten nämlich dem Versorgungsgerät (3) und dem Pflegegerät (31,32) durchlaufen soll, wobei Registrierungsmittel (16) vorgesehen sind, mittels welchen der Durchlauf oder der Sollwert und/ oder Istwert von Betriebsdaten wenigstens einer Betriebsphase registrierbar ist bzw. sind.

4. System nach Anspruch 3,
   **dadurch gekennzeichnet,**
   **dass** die Geräte ein weiteres Versorgungsgerät (3) zum Versorgen des dentalmedizinischen Instruments mit Medien umfassen.

5. System nach Anspruch 3
   **dadurch gekennzeichnet,**

**dass** die Geräte ein weiteres Pflegegerät, insbesondere ein Sterilisiergerät (31) umfassen, und wobei eines der Pflegegeräte (32) zum Reinigen und Warten des Instrumentes (2) gebildet ist.

6.  System nach Anspruch 3 bis 5,
    **dadurch gekennzeichnet,**
    **dass** eine Fehlermeldung erfolgt und/oder eine Betriebsphase an einem Gerät gesperrt wird, wenn eine vorherige Betriebsphase nicht durchlaufen worden ist oder der Istwert der Daten einer vorherigen Betriebsphase fehlt oder nicht eingehalten worden ist.

**Claims**

1.  A system for using and maintaining a medical or dental instrument (2), having a supply device (3), from which the instrument (2) is supplied with media for use, and having a maintenance device (31; 32) for maintaining the instrument (2), wherein the supply device (3) is connected to a computer (14) which is in data exchange with the supply device (3),
    and wherein both the supply device (3) and the maintenance device (31; 32) have a reading device (10b) for reading out identification and/or operatingparameter data stored in the instrument (2), **characterised in that**
    the maintenance device (31; 32) is also connected to and is in data exchange with the computer (14), and the maintenance device has a coupling portion by means of which the instrument (2) can be coupled to the maintenance device (31; 32), wherein the reading device (10b) is arranged in the region of the coupling portion.

2.  A system according to claim 1,
    **characterised in that**
    the coupling portion is formed by a protruding coupling pin (8b1), and the reading device (10b) is arranged in the coupling pin (8b1) or behind an annular stepped face from which the coupling pin (8b1) projects.

3.  A system according to claim 1 or 2,
    **characterised in that**
    the instrument (2) is to run through operating phases on at least two devices, namely the supply device (3) and the maintenance device (31; 32), wherein recording means (16) are provided by means of which the run-through or the desired value and/or actual value of operating data of at least one operating phase can be recorded.

4.  A system according to claim 3,
    **characterised in that**
    the devices include a further supply device (3) for supplying the dental instrument with media.

5.  A system according to claim 3,
    **characterised in that**
    the devices include a further maintenance device, in particular a sterilizing device (31), and wherein one of the maintenance devices (32) is formed for cleaning and servicing the instrument (2).

6.  A system according to claim 3 to 5,
    **characterised in that**
    an error message ensues and/or an operating phase at a device is blocked when a previous operating phase has not been run through or the actual value of the data of a previous operating phase is missing or has not been observed.

**Revendications**

1.  Système pour l'utilisation et le soin d'un instrument médical ou de médecine dentaire (2) comportant un appareil d'alimentation (3) par lequel l'instrument (2) est alimenté en milieux pour l'utilisation, et comportant un appareil de soin (31 ; 32) pour le soin de l'instrument (2), dans lequel l'appareil d'alimentation (3) est relié à un ordinateur (14) qui a une relation d'échange de données avec l'appareil d'alimentation (3),
    et dans lequel à la fois l'appareil d'alimentation (3) et l'appareil de soin (31 ; 32) présentent un dispositif de lecture (10b) pour lire les données de paramétrage d'identification et/ou de fonctionnement enregistrées dans l'instrument (2),
    **caractérisé en ce que**
    également l'appareil de soin (31 ; 32) est relié à l'ordinateur (14) et est en relation d'échange de données avec celui-ci,
    et **en ce que** l'appareil de soin présente une partie de couplage par laquelle l'instrument (2) peut être couplé avec l'appareil de soin (31 ; 32), dans lequel le dispositif de lecture (10b) est disposé dans la zone de la partie de couplage.

2.  Système selon la revendication 1,
    **caractérisé en ce que**
    la partie de couplage est formée par un tenon de couplage en saillie (8b1) et le dispositif de lecture (10b) est disposé dans le tenon de couplage (8b1) ou à l'arrière d'une surface à gradins annulaire, de laquelle dépasse le tenon de couplage (8b1).

3.  Système selon la revendication 1 ou 2,
    **caractérisé en ce que**
    l'instrument (2) doit parcourir des phases de fonctionnement sur au moins deux appareils, à savoir, l'appareil d'alimentation (3) et l'appareil de soin (31, 32), dans lequel des moyens d'enregistrement (16) sont prévus, par lesquels le parcours ou la valeur théorique et/ou la valeur réelle des données de fonctionnement d'au moins une phase de fonctionnement peut ou peuvent être enregistré(es).

4. Système selon la revendication 3, **caractérisé en ce que** les appareils comprennent un autre appareil d'alimentation (3) pour alimenter en milieux l'instrument de médecine dentaire.

5. Système selon la revendication 3, **caractérisé en ce que** les appareils comprennent un autre appareil de soin, en particulier un appareil de stérilisation (31) et dans lequel l'un des appareils de soin (32) est conçu pour nettoyer et entretenir l'instrument (2).

6. Système selon une revendication 3 à 5, **caractérisé en ce que** un message d'erreur est émis et/ou une phase de fonctionnement d'un appareil est bloquée si une phase de fonctionnement précédente n'a pas été réalisée ou si la valeur réelle des données d'une phase de fonctionnement précédente est manquante ou si elle n'a pas été respectée.

Fig. 1

Fig. 2

Fig. 3

Fig. 4

Fig. 5

Fig. 6

Fig. 7

Fig. 9

Fig. 8

Fig. 12

Fig. 13

Fig. 14a

Fig. 14b

Fig. 10

Fig. 11

Fig. 15

Fig. 16

EP 2 067 451 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4410276 A1
- DE 4220522 A1
- DE 19729177 A1
- DE 19913962 A1
- WO 9806338 A2